# EUROPEAN PATENT SPECIFICATION

(11) **EP 1 987 033 B1**
(45) Date of publication and mention of the grant of the patent: **25.08.2010**
(21) Application number: 07712214.1
(22) Date of filing: 13.02.2007
(51) Int. Cl.: C07D 471/08, A61K 31/4995, A61P 25/28

(54) **DIAZABICYCLOALKANE DERIVATIVES AND THEIR MEDICAL USE**
DIAZABICYCLOALKAN-DERIVATE UND IHRE MEDIZINISCHE VERWENDUNG
DÉRIVÉS DE DIAZABICYCLOALKANE ET LEUR UTILISATION MÉDICALE

(30) Priority: 14.02.2006 DK 200600210; 15.02.2006 US 773325 P
(43) Date of publication of application: 05.11.2008
(73) Proprietor: NeuroSearch A/S, 2750 Ballerup (DK)
(72) Inventor: PETERS, Dan, DK-2750 Ballerup (DK); TIMMERMANN, Daniel, B., DK-2750 Ballerup (DK); OLSEN, Gunnar, M., DK-2750 Ballerup (DK); NIELSEN, Elsebet, Østergaard, DK-2750 Ballerup (DK); CHRISTENSEN, Jeppe, Kejser, DK-2750 Ballerup (DK); DYHRING, Tino, DK-2750 Ballerup (DK)
(74) Representative: Abildgren, Michael Padkjaer
(86) International application number: PCT/EP2007/051396
(87) International publication number: WO 2007/093600

(56) References cited:
- EP-A- 1 231 212
- EP-A- 1 359 152
- WO-A-2000/58311
- WO-A-2006/087306
- US-A1- 2003 119 837

## Description

### TECHNICAL FIELD

This invention relates to novel diaza-bicyclo-alkane derivatives, which are found to be cholinergic ligands at the nicotinic acetylcholine receptors and modulators of the monoamine receptors and transporters. Due to their pharmacological profile the compounds of the invention may be useful for the treatment of diseases or disorders as diverse as those related to the cholinergic system of the central nervous system (CNS), the peripheral nervous system (PNS), diseases or disorders related to smooth muscle contraction, endocrine diseases or disorders, diseases or disorders related to neuro-degeneration, diseases or disorders related to inflammation, pain, and withdrawal symptoms caused by the termination of abuse of chemical substances.

### BACKGROUND ART

The endogenous cholinergic neurotransmitter, acetylcholine, exert its biological effect via two types of cholinergic receptors, the muscarinic Acetyl Choline Receptors (mAChR) and the nicotinic Acetyl Choline Receptors (nAChR).

As it is well established that muscarinic acetylcholine receptors dominate quantitatively over nicotinic acetylcholine receptors in the brain area important to memory and cognition, and much research aimed at the development of agents for the treatment of memory related disorders have focused on the synthesis of muscarinic acetylcholine receptor modulators.

Recently, however, an interest in the development of nAChR modulators has emerged. Several diseases are associated with degeneration of the cholinergic system i.e. senile dementia of the Alzheimer type, vascular dementia and cognitive impairment due to the organic brain damage disease related directly to alcoholism. Indeed several CNS disorders can be attributed to a cholinergic deficiency, a dopaminergic deficiency, an adrenergic deficiency or a serotonergic deficiency.

US 2003 119837 describes certain diazabicycloalkane derivatives useful as nicotinic α7 receptor agonists. Only bicyclic heterocyclic derivatives are reported.

EP 1359152 describes certain diazabicyclic derivatives useful as nicotinic acetylcholine receptor ligands. Various monocyclic and bicyclic heterocyclic derivatives are reported.

WO 2000 58311 and EP 1231212 describe certain diazabicycloalkane derivatives, including 1,4-Diaza-bicyclo[3.2.2]nonane-4-carboxylic acid 4-benzyl-phenyl ester and 1,4-Diaza-bicyclo[3.2.2]nonane-4-carboxylic acid 4-phenoxy-phenyl ester, useful as nicotinic α7 receptor agonists.

### SUMMARY OF THE INVENTION

The present invention is devoted to the provision novel modulators of the nicotinic and/or of the monoamine receptors, which modulators are useful for the treatment of diseases or disorders related to the cholinergic receptors, and in particular the nicotinic acetylcholine receptor (nAChR), the serotonin receptor (5-HTR), the dopamine receptor (DAR) and the norepinephrine receptor (NER), and of the biogenic amine transporters for serotonin (5-HT), dopamine (DA) and norepinephrine (NE).

In its first aspect the invention provides novel diaza-bicyclo-alkane derivatives represented by Formula I 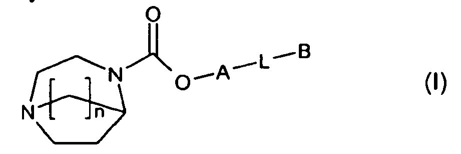 an isomer thereof or a mixture of its isomers, or a pharmaceutically acceptable salt thereof, wherein
n is 1, 2 or 3;
A represents a phenyl, thiadiazolyl, pyridyl or pyridazinyl group;
B represents a phenyl or an indolyl group; which phenyl and indolyl group may optionally be substituted with hydroxy or alkoxy; and
L represents a linking group selected from -C≡C- and -NHCO-.

In its second aspect the invention provides pharmaceutical compositions comprising a therapeutically effective amount of the diazabicyclic aryl derivative of the invention, or a pharmaceutically acceptable addition salt thereof, together with at least one pharmaceutically acceptable carrier or diluent.

In a further aspect the invention relates to the use of the diazabicyclic aryl derivative of the invention, or a pharmaceutically acceptable addition salt thereof, for the manufacture of a pharmaceutical composition/medicament for the treatment, prevention or alleviation of a disease or a disorder or a condition of a mammal, including a human, which disease, disorder or condition is responsive to modulation of cholinergic receptors and/or monoamine receptors.

Other objects of the invention will be apparent to the person skilled in the art from the following detailed description and examples.

### DETAILED DISCLOSURE OF THE INVENTION

### Diazabicyclic Aryl Derivative

In a first aspect novel diaza-bicyclo-alkane derivatives are provided. The diaza-bicyclo-alkane derivatives of the invention may be represented by Formula I 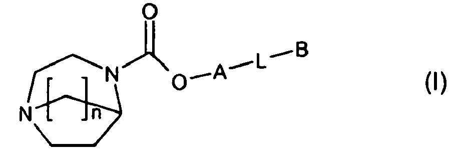 an isomer thereof or a mixture of its isomers, or a pharmaceutically acceptable salt thereof, wherein
n is 1, 2 or 3;
A represents a phenyl, thiadiazolyl, pyridyl or pyridazinyl group;
B represents a phenyl or an indolyl group; which phenyl and indolyl group may optionally be substituted with hydroxy or alkoxy; and
L represents a linking group selected from -C=C- and -NHCO-.

In a preferred embodiment the diaza-bicyclo-alkane derivative of the invention is a compound of Formula I, wherein n is 1 or 2.

In a more preferred embodiment n is 2.

In another preferred embodiment the diaza-bicyclo-alkane derivative of the invention is a compound of Formula I, wherein A represents a phenyl group.

In a third preferred embodiment B represents a phenyl group; which phenyl may optionally be substituted with hydroxy or alkoxy, in particular methoxy or ethoxy.

In a fourth preferred embodiment B represents an indolyl group, in particular a 1*H*-indol-2-yl, 1*H*-indol-5-yl or 1*H*-indol-6-yl.

In a still further more preferred embodiment B represents a phenyl group.

In a fifth more preferred embodiment L represents -C=C- or -NHCO-.

In a most preferred embodiment the diaza-bicyclo-alkane derivative of the invention is
1,4-Diaza-bicyclo[3.2.2]nonane-4-carboxylic acid 4-phenylethynyl-phenyl ester;
1,4-Diaza-bicyclo[3.2.2]nonane-4-carboxylic acid 4-benzoylamino-phenyl ester;
1,4-Diaza-bicyclo[3.2.2]nonane-4-carboxylic acid 4-(4-methoxy-benzoylamino)-phenyl ester;
1,4-Diaza-bicyclo[3.2.2]nonane-4-carboxylic acid 4-(3-methoxy-benzoylamino)-phenyl ester; or
1,4-Diaza-bicyclo[3.2.2]nonane-4-carboxylic acid 4-(2-methoxy-benzoylamino)-phenyl ester;
or an enantiomers or a mixture of its enantiomers, or a pharmaceutically acceptable salt thereof.

Any combination of two or more of the embodiments described herein is considered within the scope of the present invention.

### Definition of Substituents

In the context of this invention an alkyl group designates a univalent saturated, straight or branched hydrocarbon chain. The hydrocarbon chain preferably contain of from one to eighteen carbon atoms (C₁₋₁₈-alkyl), more preferred of from one to six carbon atoms (C₁₋₆-alkyl; lower alkyl), including pentyl, isopentyl, neopentyl, tertiary pentyl, hexyl and isohexyl. In a preferred embodiment alkyl represents a C₁₋₄-alkyl group, including butyl, isobutyl, secondary butyl, and tertiary butyl. In another preferred embodiment of this invention alkyl represents a C₁₋₃-alkyl group, which may in particular be methyl, ethyl, propyl or isopropyl.

In the context of this invention an alkoxy group designates an "alkyl-O-" group, wherein alkyl is as defined above. Examples of preferred alkoxy groups of the invention include methoxy and ethoxy.

### Pharmaceutically Acceptable Salts

The diazabicyclic aryl derivative of the invention may be provided in any form suitable for the intended administration. Suitable forms include pharmaceutically (i.e. physiologically) acceptable salts, and pre- or prodrug forms of the chemical compound of the invention.

Examples of pharmaceutically acceptable addition salts include, without limitation, the non-toxic inorganic and organic acid addition salts such as the hydrochloride derived from hydrochloric acid, the hydrobromide derived from hydrobromic acid, the nitrate derived from nitric acid, the perchlorate derived from perchloric acid, the phosphate derived from phosphoric acid, the sulphate derived from sulphuric acid, the formate derived from formic acid, the acetate derived from acetic acid, the aconate derived from aconitic acid, the ascorbate derived from ascorbic acid, the benzenesulphonate derived from benzensulphonic acid, the benzoate derived from benzoic acid, the cinnamate derived from cinnamic acid, the citrate derived from citric acid, the embonate derived from embonic acid, the enantate derived from enanthic acid, the fumarate derived from fumaric acid, the glutamate derived from glutamic acid, the glycolate derived from glycolic acid, the lactate derived from lactic acid, the maleate derived from maleic acid, the malonate derived from malonic acid, the mandelate derived from mandelic acid, the methanesulphonate derived from methane sulphonic acid, the naphthalene-2-sulphonate derived from naphtalene-2-sulphonic acid, the phthalate derived from phthalic acid, the salicylate derived from salicylic acid, the sorbate derived from sorbic acid, the stearate derived from stearic acid, the succinate derived from succinic acid, the tartrate derived from tartaric acid, the toluene-p-sulphonate derived from p-toluene sulphonic acid, and the like. Such salts may be formed by procedures well known and described in the art.

Other acids such as oxalic acid, which may not be considered pharmaceutically acceptable, may be useful in the preparation of salts useful as intermediates in obtaining a chemical compound of the invention and its pharmaceutically acceptable acid addition salt.

Additional examples of pharmaceutically acceptable addition salts include, without limitation, the non-toxic inorganic and organic acid addition salts such as the hydrochloride, the hydrobromide, the nitrate, the perchlorate, the phosphate, the sulphate, the formate, the acetate, the aconate, the ascorbate, the benzenesulphonate, the benzoate, the cinnamate, the citrate, the embonate, the enantate, the fumarate, the glutamate, the glycolate, the lactate, the maleate, the malonate, the mandelate, the methanesulphonate, the naphthalene-2-sulphonate derived, the phthalate, the salicylate, the sorbate, the stearate, the succinate, the tartrate, the toluene-p-sulphonate, and the like. Such salts may be formed by procedures well known and described in the art.

Metal salts of a chemical compound of the invention include alkali metal salts, such as the sodium salt of a chemical compound of the invention containing a carboxy group.

In the context of this invention the "onium salts" of N-containing compounds are also contemplated as pharmaceutically acceptable salts. Preferred "onium salts" include the alkyl-onium salts, the cycloalkyl-onium salts, and the cycloalkylalkyl-onium salts.

### Isomers

It will be appreciated by those skilled in the art that the compounds of the present invention may exist in different stereoisomeric forms, including enantiomers, diastereomers, as well as geometric isomers (cis-trans isomers). The invention includes all such isomers and any mixtures thereof including racemic mixtures.

Racemic forms can be resolved into the optical antipodes by known methods and techniques. One way of separating the isomeric salts is by use of an optically active acid, and liberating the optically active amine compound by treatment with a base. Another method for resolving racemates into the optical antipodes is based upon chromatography on an optical active matrix. Racemic compounds of the present invention can thus be resolved into their optical antipodes, e.g., by fractional crystallisation of d- or I- (tartrates, mandelates, or camphorsulphonate) salts for example.

The chemical compounds of the present invention may also be resolved by the formation of diastereomeric amides by reaction of the chemical compounds of the present invention with an optically active activated carboxylic acid such as that derived from (+) or (-) phenylalanine, (+) or (-) phenylglycine, (+) or (-) camphanic acid or by the formation of diastereomeric carbamates by reaction of the chemical compound of the present invention with an optically active chloroformate or the like.

Additional methods for the resolving the optical isomers are known in the art. Such methods include those described by Jaques J, Collet A, & Wilen S in "Enantiomers, Racemates, and Resolutions", John Wiley and Sons, New York (1981).

Optical active compounds can also be prepared from optical active starting materials.

### Labelled Compounds

The compounds of the invention may be used in their labelled or unlabelled form. In the context of this invention the labelled compound has one or more atoms replaced by an atom having an atomic mass or mass number different from the atomic mass or mass number usually found in nature. The labelling will allow easy quantitative detection of said compound.

The labelled compounds of the invention may be useful as diagnostic tools, radio tracers, or monitoring agents in various diagnostic methods, and for in vivo receptor imaging.

The labelled isomer of the invention preferably contains at least one radionuclide as a label. Positron emitting radionuclides are all candidates for usage. In the context of this invention the radionuclide is preferably selected from ²H (deuterium), ³H (tritium), ¹³C, ¹⁴C, ¹³¹I, ¹²⁵I, ¹²³I, and ¹⁸F.

The physical method for detecting the labelled isomer of the present invention may be selected from Position Emission Tomography (PET), Single Photon Imaging Computed Tomography (SPECT), Magnetic Resonance Spectroscopy (MRS), Magnetic Resonance Imaging (MRI), and Computed Axial X-ray Tomography (CAT), or combinations thereof.

### Methods of Producing Diazabicyclic Aryl Derivatives

The diazabicyclic aryl derivative of the invention may be prepared by conventional methods for chemical synthesis, e.g. those described in the working examples. The starting materials for the processes described in the present application are known or may readily, be prepared by conventional methods from commercially available chemicals.

Also one compound of the invention can be converted to another compound of the invention using conventional methods.

The end products of the reactions described herein may be isolated by conventional techniques, e.g. by extraction, crystallisation, distillation, chromatography, etc.

### Biological Activity

The present invention is devoted to the provision novel ligands and modulators of the nicotinic receptors, which ligands and modulators are useful for the treatment of diseases or disorders related to the cholinergic receptors, and in particular the nicotinic acetylcholine receptor (nAChR). Preferred compounds of the invention show a pronounced nicotinic acetylcholine α7 receptor subtype selectivity.

Due to their pharmacological profile the compounds of the invention may be useful for the treatment of diseases or conditions as diverse as CNS related diseases, PNS related diseases, diseases related to smooth muscle contraction, endocrine disorders, diseases related to neuro-degeneration, diseases related to inflammation, pain, and withdrawal symptoms caused by the termination of abuse of chemical substances.

In a preferred embodiment the compounds of the present invention may be useful for the treatment, prevention or alleviation of a cognitive disorder, learning deficit, memory deficits and dysfunction, Alzheimer's disease, attention deficit, attention deficit hyperactivity disorder (ADHD), Tourette's syndrome, psychosis, depression, Bipolar Disorder, mania, manic depression, schizophrenia, cognitive or attention deficits related to schizophrenia, obsessive compulsive disorders (OCD), panic disorders, eating disorders such as anorexia nervosa, bulimia and obesity, narcolepsy, nociception, AIDS-dementia, senile dementia, autism, Parkinson's disease, Huntington's disease, Amyotrophic Lateral Sclerosis, anxiety, non-OCD anxiety disorders, convulsive disorders, epilepsy, neurodegenerative disorders, transient anoxia, induced neuro-degeneration, neuropathy, diabetic neuropathy, periferic dyslexia, tardive dyskinesia, hyperkinesia, mild pain, moderate or severe pain, pain of acute, chronic or recurrent character, pain caused by migraine, postoperative pain, phantom limb pain, inflammatory pain, neuropathic pain, chronic headache, central pain, pain related to diabetic neuropathy, to post therapeutic neuralgia, or to peripheral nerve injury, bulimia, post-traumatic syndrome, social phobia, sleeping disorders, pseudodementia, Ganser's syndrome, pre-menstrual syndrome, late luteal phase syndrome, chronic fatigue syndrome, mutism, trichotillomania, jet-lag, arrhythmias, smooth muscle contractions, angina pectoris, premature labour, diarrhoea, asthma, tardive dyskinesia, hyperkinesia, premature ejaculation, erectile difficulty, hypertension, inflammatory disorders, inflammatory skin disorders, acne, rosacea, Crohn's disease, inflammatory bowel disease, ulcerative colitis, diarrhoea, or withdrawal symptoms caused by termination of use of addictive substances, including nicotine containing products such as tobacco, opioids such as heroin, cocaine and morphine, benzodiazepines and benzodiazepine-like drugs, and alcohol.

In a more preferred embodiment the compounds of the invention may be useful for the treatment, prevention or alleviation of pain, mild or moderate or severe pain, pain of acute, chronic or recurrent character, pain caused by migraine, postoperative pain, phantom limb pain, inflammatory pain, neuropathic pain, chronic headache, central pain, pain related to diabetic neuropathy, to post therapeutic neuralgia, or to peripheral nerve injury.

In an even more preferred embodiment the compounds of the invention may be useful for the treatment, prevention or alleviation of diseases, disorders or conditions associated with smooth muscle contractions, convulsive disorders, angina pectoris, premature labour, convulsions, diarrhoea, asthma, epilepsy, tardive dyskinesia, hyperkinesia, premature ejaculation, or erectile difficulty.

In a still more preferred embodiment the compounds of the invention may be useful for the treatment, prevention or alleviation of a neurodegenerative disorder, transient anoxia, or induced neuro-degeneration.

In a yet more preferred embodiment the compounds of the invention may be useful for the treatment, prevention or alleviation of an inflammatory disorder, inflammatory skin disorder, acne, rosacea, Crohn's disease, inflammatory bowel disease, ulcerative colitis, or diarrhoea.

In a further preferred embodiment the compounds of the invention may be useful for the treatment, prevention or alleviation of diabetic neuropathy, schizophrenia, cognitive or attentional deficits related to schizophrenia, or depression.

Finally the compounds of the invention may be useful for the treatment of withdrawal symptoms caused by termination of use of addictive substances. Such addictive substances include nicotine containing products such as tobacco, opioids such as heroin, cocaine and morphine, benzodiazepines, benzodiazepine-like drugs, and alcohol. Withdrawal from addictive substances is in general a traumatic experience characterised by anxiety and frustration, anger, anxiety, difficulties in concentrating, restlessness, decreased heart rate and increased appetite and weight gain.

In this context "treatment" covers treatment, prevention, prophylactics and alleviation of withdrawal symptoms and abstinence as well as treatment resulting in a voluntary diminished intake of the addictive substance.

In another aspect, the compounds of the invention are used as diagnostic agents, e.g. for the identification and localisation of nicotinic receptors in various tissues.

It is at present contemplated that a suitable dosage of the active pharmaceutical ingredient (API) is within the range of from about 0.1 to about 1000 mg API per day, more preferred of from about 10 to about 500 mg API per day, most preferred of from about 30 to about 100 mg API per day, dependent, however, upon the exact mode of administration, the form in which it is administered, the indication considered, the subject and in particular the body weight of the subject involved, and further the preference and experience of the physician or veterinarian in charge.

Preferred compounds of the invention show a biological activity in the sub-micromolar and micromolar range, i.e. of from below 1 to about 100 µM.

### Pharmaceutical Compositions

In another aspect the invention provides novel pharmaceutical compositions comprising a therapeutically effective amount of diazabicyclic aryl derivative of the invention.

While a chemical compound of the invention for use in therapy may be administered in the form of the raw chemical compound, it is preferred to introduce the active ingredient, optionally in the form of a physiologically acceptable salt, in a pharmaceutical composition together with one or more adjuvants, excipients, carriers, buffers, diluents, and/or other customary pharmaceutical auxiliaries.

In a preferred embodiment, the invention provides pharmaceutical compositions comprising the diazabicyclic aryl derivative of the invention, or a pharmaceutically acceptable salt or derivative thereof, together with one or more pharmaceutically acceptable carriers therefore, and, optionally, other therapeutic and/or prophylactic ingredients, known and used in the art. The carrier(s) must be "acceptable" in the sense of being compatible with the other ingredients of the formulation and not harmful to the recipient thereof.

The pharmaceutical composition of the invention may be administered by any convenient route, which suits the desired therapy. Preferred routes of administration include oral administration, in particular in tablet, in capsule, in dragé, in powder, or in liquid form, and parenteral administration, in particular cutaneous, subcutaneous, intramuscular, or intravenous injection. The pharmaceutical composition of the invention can be manufactured by any skilled person by use of standard methods and conventional techniques appropriate to the desired formulation. When desired, compositions adapted to give sustained release of the active ingredient may be employed.

Pharmaceutical compositions of the invention may be those suitable for oral, rectal, bronchial, nasal, pulmonal, topical (including buccal and sub-lingual), transdermal, vaginal or parenteral (including cutaneous, subcutaneous, intramuscular, intraperitoneal, intravenous, intraarterial, intracerebral, intraocular injection or infusion) administration, or those in a form suitable for administration by inhalation or insufflation, including powders and liquid aerosol administration, or by sustained release systems. Suitable examples of sustained release systems include semipermeable matrices of solid hydrophobic polymers containing the compound of the invention, which matrices may be in form of shaped articles, e.g. films or microcapsules.

The chemical compound of the invention, together with a conventional adjuvant, carrier, or diluent, may thus be placed into the form of pharmaceutical compositions and unit dosages thereof. Such forms include solids, and in particular tablets, filled capsules, powder and pellet forms, and liquids, in particular aqueous or non-aqueous solutions, suspensions, emulsions, elixirs, and capsules filled with the same, all for oral use, suppositories for rectal administration, and sterile injectable solutions for parenteral use. Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed.

The chemical compound of the present invention can be administered in a wide variety of oral and parenteral dosage forms. It will be obvious to those skilled in the art that the following dosage forms may comprise, as the active component, either a chemical compound of the invention or a pharmaceutically acceptable salt of a chemical compound of the invention.

For preparing pharmaceutical compositions from a chemical compound of the present invention, pharmaceutically acceptable carriers can be either solid or liquid. Solid form preparations include powders, tablets, pills, capsules, cachets, suppositories, and dispersible granules. A solid carrier can be one or more substances which may also act as diluents, flavouring agents, solubilizers, lubricants, suspending agents, binders, preservatives, tablet disintegrating agents, or an encapsulating material.

In powders, the carrier is a finely divided solid, which is in a mixture with the finely divided active component.

In tablets, the active component is mixed with the carrier having the necessary binding capacity in suitable proportions and compacted in the shape and size desired.

The powders and tablets preferably contain from five or ten to about seventy percent of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

For preparing suppositories, a low melting wax, such as a mixture of fatty acid glyceride or cocoa butter, is first melted and the active component is dispersed homogeneously therein, as by stirring. The molten homogenous mixture is then poured into convenient sized moulds, allowed to cool, and thereby to solidify.

Compositions suitable for vaginal administration may be presented as pessaries, tampons, creams, gels, pastes, foams or sprays containing in addition to the active ingredient such carriers as are known in the art to be appropriate.

Liquid preparations include solutions, suspensions, and emulsions, for example, water or water-propylene glycol solutions. For example, parenteral injection liquid preparations can be formulated as solutions in aqueous polyethylene glycol solution.

The chemical compound according to the present invention may thus be formulated for parenteral administration (e.g. by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, and may contain formulation agents such as suspending, stabilising and/or dispersing agents. Alternatively, the active ingredient may be in powder form, obtained by aseptic isolation of sterile solid or by lyophilization from solution, for constitution with a suitable vehicle, e.g. sterile, pyrogen-free water, before use.

Aqueous solutions suitable for oral use can be prepared by dissolving the active component in water and adding suitable colorants, flavours, stabilising and thickening agents, as desired.

Aqueous suspensions suitable for oral use can be made by dispersing the finely divided active component in water with viscous material, such as natural or synthetic gums, resins, methylcellulose, sodium carboxymethylcellulose, or other well known suspending agents.

Also included are solid form preparations, intended for conversion shortly before use to liquid form preparations for oral administration. Such liquid forms include solutions, suspensions, and emulsions. In addition to the active component such preparations may comprise colorants, flavours, stabilisers, buffers, artificial and natural sweeteners, dispersants, thickeners, solubilizing agents, and the like.

For topical administration to the epidermis the chemical compound of the invention may be formulated as ointments, creams or lotions, or as a transdermal patch. Ointments and creams may, for example, be formulated with an aqueous or oily base with the addition of suitable thickening and/or gelling agents. Lotions may be formulated with an aqueous or oily base and will in general also contain one or more emulsifying agents, stabilising agents, dispersing agents, suspending agents, thickening agents, or colouring agents.

Compositions suitable for topical administration in the mouth include lozenges comprising the active agent in a flavoured base, usually sucrose and acacia or tragacanth; pastilles comprising the active ingredient in an inert base such as gelatin and glycerine or sucrose and acacia; and mouthwashes comprising the active ingredient in a suitable liquid carrier.

Solutions or suspensions are applied directly to the nasal cavity by conventional means, for example with a dropper, pipette or spray. The compositions may be provided in single or multi-dose form.

Administration to the respiratory tract may also be achieved by means of an aerosol formulation in which the active ingredient is provided in a pressurised pack with a suitable propellant such as a chlorofluorocarbon (CFC) for example dichlorodifluoromethane, trichlorofluoromethane, or dichlorotetrafluoroethane, carbon dioxide, or other suitable gas. The aerosol may conveniently also contain a surfactant such as lecithin. The dose of drug may be controlled by provision of a metered valve.

Alternatively the active ingredients may be provided in the form of a dry powder, for example a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity. The powder composition may be presented in unit dose form for example in capsules or cartridges of, e.g., gelatin, or blister packs from which the powder may be administered by means of an inhaler.

In compositions intended for administration to the respiratory tract, including intranasal compositions, the compound will generally have a small particle size for example of the order of 5 microns or less. Such a particle size may be obtained by means known in the art, for example by micronization.

When desired, compositions adapted to give sustained release of the active ingredient may be employed.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packaged tablets, capsules, and powders in vials or ampoules. Also, the unit dosage form can be a capsule, tablet, cachet, or lozenge itself, or it can be the appropriate number of any of these in packaged form.

Tablets or capsules for oral administration and liquids for intravenous administration and continuous infusion are preferred compositions.

Further details on techniques for formulation and administration may be found in the latest edition of Remington's Pharmaceutical Sciences (Maack Publishing Co., Easton, PA).

The actual dosage depends on the nature and severity of the disease being treated, and is within the discretion of the physician, and may be varied by titration of the dosage to the particular circumstances of this invention to produce the desired therapeutic effect. However, it is presently contemplated that pharmaceutical compositions containing of from about 0.1 to about 500 mg of active ingredient per individual dose, preferably of from about 1 to about 100 mg, most preferred of from about 1 to about 10 mg, are suitable for therapeutic treatments.

The active ingredient may be administered in one or several doses per day. A satisfactory result can, in certain instances, be obtained at a dosage as low as 0.1 µg/kg i.v. and 1 µg/kg p.o. The upper limit of the dosage range is presently considered to be about 10 mg/kg i.v. and 100 mg/kg p.o. Preferred ranges are from about 0.1 µg/kg to about 10 mg/kg/day i.v., and from about 1 µg/kg to about 100 mg/kg/day p.o.

### EXAMPLES

The invention is further illustrated with reference to the following examples.

### Example 1

### Preparatory Example

All reactions involving air sensitive reagents or intermediates were performed under nitrogen and in anhydrous solvents. Magnesium sulphate was used as drying agent in the workup-procedures and solvents were evaporated under reduced pressure.

### Method A

### 1,4-Diaza-bicyclo[3.2.2]nonane-4-carboxylic acid 4-phenylethynyl-phenyl ester fumaric acid salt (Compound A1)

A mixture of 1,4-diaza-bicyclo[3.2.2]nonane-4-carboxylic acid 4-bromophenyl ester hydrochloric acid salt (2.25 g, 6.22 mmol), converted to the corresponding free base, phenylacetylene (14.6 g, 143 mmol), palladacycle (0.23 g, 0.25 mmol), diisopropylethylamine (3.2 g, 24.8 mmol), copper iodide (0.24 g, 1.24 mmol) and tetrakistriphenylphosphinepalladium(0) (0.12 g, 0.104 mmol) and dioxane (40 ml) was stirred at reflux for 4 weeks. The mixture was allowed to cool to room temperature. Aqueous sodium hydroxide (100 ml, 1M) was added followed by extraction with dichloromethane (3 x 50 ml). The crude mixture was repeatedly purified four times by silica gel column chromatography by using a mixture of dichloromethane, methanol and aqueous ammonia (9:1 + 1 %). The corresponding salt was obtained by addition of a diethyl ether and methanol mixture (9:1) saturated with fumaric acid. Yield 190 mg (6.6%). Mp. 160-177°C.

### Method B

### 1,4-diaza-bicyclo[3.2.2]nonane-4-carboxylic acid 4-bromo-phenyl ester hydrochloric acid salt (Intermediate compound)

1,4-Diaza-bicyclo[3.2.2]nonane (5.49 g, 43.5 mmol), obtained according to literature, was slowly added to a mixture of 4-bromophenyl chloroformate (10.25 g, 43.5 mmol) and 1,2-dimethoxyethane (100 ml). The mixture was allowed to stir at room temperature for 15 hours. The precipitated product was filtered and washed with 1,2-dimethoxyethane. Yield 11.3 g (72%). Mp. 242°C.

### Method C

### 1.4-Diaza-bicyclo[3.2.2]nonane-4-carboxylic acid 4-benzoylamino-phenyl ester hydrochloric acid salt (Compound C1)

A mixture of 1,4-diaza-bicyclo[3.2.2]nonane-4-carboxylic acid 4-aminophenyl ester (2.6 g, 8.56 mmol), benzoyl chloride (1.40 g, 8.56 mmol) and ethanol (40 ml) was stirred for 15 hours. Diethylether (100 ml) was added and the mixture was stirred and filtered. The solid (2.5 g) was recrystallised from ethanol. Yield 1.15 g (33%). Mp. > 270°C. LC-ESI-HRMS of [M+H]+ shows 366.1804 Da. Calc. 366.181767 Da, dev. -3.7 ppm.

### 1,4-Diaza-bicyclo[3.2.2]nonane-4-carboxylic acid 4-(4-methoxy-benzoylamino)-phenyl ester hydrochloric acid salt (Compound C2)

Prepared according to Method C. LC-ESI-HRMS of [M+H]+ shows 396.1931 Da. Calc. 396.192332 Da, dev. 1.9 ppm.

### 1,4-Diaza-bicyclo[3.2.2]nonane-4-carboxylic acid 4-(3-methoxy-benzoylamino)-phenyl ester hydrochloric acid salt (Compound C3)

Prepared according to Method C. LC-ESI-HRMS of [M+H]+ shows 396.1935 Da. Calc. 396.192332 Da, dev. 2.9 ppm.

### 1,4-Diaza-bicyclo[3.2.2]nonane-4-carboxylic acid 4-(2-methoxy-benzoylamino)-phenyl ester hydrochloric acid salt (Compound C4)

Prepared according to Method C LC-ESI-HRMS of [M+H]+ shows 396.1917 Da. Calc. 396.192332 Da, dev. -1.6 ppm.

### 1,4-Diaza-bicyclo[3.2.2]nonane-4-carboxylic acid 4-amino-phen ester hydrochloric acid salt salt (Intermediate compound)

A mixture of 1,4-diaza-bicyclo[3.2.2]nonane-4-carboxylic acid 4-nitro-phenyl ester hydrochloric acid salt (2.9 g, 8.85 mmol), 2-propanol (100 ml) and palladium 10% on activated carbon (500 mg) was stirred under an atmosphere of hydrogen for 30 minutes. The mixture was filtered through celite followed by extraction with methanol (100 ml). The crude mixture was evaporated. Yield 2.6 g (99%).

### 1,4-Diaza-bicyclo[3.2.2]nonane-4-carboxylic acid 4-nitro-phenyl ester hydrochloric acid salt (Intermediate compound)

A mixture of 1,4-diaza-bicyclo[3.2.2]nonane (6.31 g, 50 mmol) and 1,2-dimethoxyethane (40 ml) was added to a mixture of 4-nitrophenylchloroformate (10.1 g, 50 mmol) and 1,2-dimethoxyethane (100 ml) at room temperature. The mixture was stirred at room temperature for 21 hours. The precipitated solid was filtered and was purified by stirring in 1,2-dimethoxyethane (100 ml) followed by filtration. Yield 12.3 g (75%).

### Example 2

### In vitro Inhibition of ³H-α-Bungarotoxine Binding in Rat Brain

In this example the affinity of the compounds of the invention for binding to α₇-subtype of nicotinic receptors is determined.

α-Bungarotoxine is a peptide isolated from the venom of the Elapidae snake *Bungarus multicinctus.* It has high affinity for neuronal and neuromuscular nicotinic receptors, where it acts as a potent antagonist. ³H-α-Bungarotoxine labels nicotinic acetylcholine receptors formed by the α₇ subunit isoform found in brain and the α₁ isoform in the neuromuscular junction.

### Tissue preparation

Preparations are performed at 0-4°C. Cerebral cortices from male Wistar rats (150-250 g) are homogenised for 10 seconds in 15 ml of 20 mM Hepes buffer containing 118 mM NaCl, 4.8 mM KCl, 1.2 mM MgSO₄ and 2.5 mM CaCl₂ (pH 7.5) using an Ultra-Turrax homogeniser. The tissue suspension is subjected to centrifugation at 27,000 x g for 10 minutes. The supernatant is discarded and the pellet is washed twice by centrifugation at 27,000 x g for 10 minutes in 20 ml of fresh buffer, and the final pellet is then re-suspended in fresh buffer containing 0.01 % BSA (35 ml per g of original tissue) and used for binding assays.

### Assay

Aliquots of 500 µl of homogenate are added to 25 µl of test solution and 25 µl of ³H-α-bungarotoxine (2 nM, final concentration) and mixed and incubated for 2 hours at 37°C. Non-specific binding is determined using (-)-nicotine (1 mM, final concentration). After incubation, the samples are added 5 ml of ice-cold Hepes buffer containing 0.05% PEI and poured directly onto Whatman GF/C glass fibre filters (pre-soaked in 0.1% PEI for at least 6 hours) under suction, and immediately washed with 2 x 5 ml ice-cold buffer.

The amount of radioactivity on the filters is determined by conventional liquid scintillation counting. Specific binding is total binding minus non-specific binding.

The test value is given as an IC₅₀ (the concentration of the test substance which inhibits the specific binding of ³H-α-bungarotoxin by 50%).

The results of these experiments are presented in Table 1 below.

**Table 1**

| Inhibition of ³H-α-Bungarotoxine Binding | |
|---|---|
| **Coumpound No.** | **IC₅₀ (µM)** |
| A1 | 0,42 |
| C1 | 0.56 |

## Claims

1. A diaza-bicyclo-alkane derivative represented by Formula I 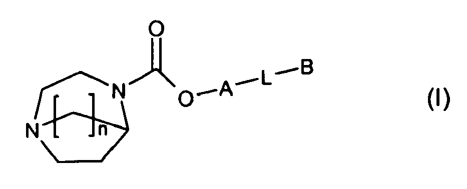 an isomer thereof or a mixture of its isomers, or a pharmaceutically acceptable salt thereof, wherein
n is 1, 2 or 3;
A represents a phenyl, thiadiazolyl, pyridyl or pyridazinyl group;
B represents a phenyl or an indolyl group; which phenyl and indolyl group may optionally be substituted with hydroxy or alkoxy; and
L represents a linking group selected from -C=C- and -NHCO-.

2. The diaza-bicyclo-alkane derivative of claim 1, wherein n is 1 or 2.

3. The diaza-bicyclo-alkane derivative of claim 1, wherein n is 2.

4. The diaza-bicyclo-alkane derivative of claim 3, wherein A represents a phenyl group.

5. The diaza-bicyclo-alkane derivative of any one of claims 1-4, wherein B represents a phenyl group; which phenyl may optionally be substituted with hydroxy or alkoxy.

6. The diaza-bicyclo-alkane derivative of any one of claims 1-4, wherein B represents B represents an indolyl group.

7. The diaza-bicyclo-alkane derivative of any one of claims 1-6, wherein L represents -C≡C-.

8. The diaza-bicyclo-alkane derivative of any one of claims 1-7, wherein L represents -NHCO-.

9. The diaza-bicyclo-alkane derivative of claim 1, which is
1,4-Diaza-bicyclo[3.2.2]nonane-4-carboxylic acid 4-phenylethynyl-phenyl ester;
1,4-Diaza-bicyclo[3.2.2]nonane-4-carboxylic acid 4-benzoylamino-phenyl ester;
1,4-Diaza-bicyclo[3.2.2]nonane-4-carboxylic acid 4-(4-methoxy-benzoylamino)-phenyl ester;
1,4-Diaza-bicyclo[3.2.2]nonane-4-carboxylic acid 4-(3-methoxy-benzoylamino)-phenyl ester; or
1,4-Diaza-bicyclo[3.2.2]nonane-4-carboxylic acid 4-(2-methoxy-benzoylamino)-phenyl ester;
or an enantiomers or a mixture of its enantiomers, or a pharmaceutically acceptable salt thereof.

10. A pharmaceutical composition comprising a therapeutically effective amount of a diaza-bicyclo-alkane derivative of any one of claims 1-9, or a pharmaceutically acceptable addition salt thereof, together with at least one pharmaceutically acceptable carrier or diluent.

11. Use of a diaza-bicyclo-alkane derivative of any one of claims 1-9, or a pharmaceutically acceptable addition salt thereof, for the manufacture of a pharmaceutical composition/medicament for the treatment, prevention or alleviation of a disease or a disorder or a condition of a mammal, including a human, which disease, disorder or condition is responsive to modulation of cholinergic receptors and/or monoamine receptors.

12. The use according to claim 11, wherein the disease or a disorder or a condition is a cognitive disorder, a learning deficit, a memory deficit or dysfunction, Alzheimer's disease, attention deficit, attention deficit hyperactivity disorder (ADHD), Tourette's syndrome, psychosis, depression, Bipolar Disorder, mania, manic depression, schizophrenia, cognitive or attention deficits related to schizophrenia, obsessive compulsive disorders (OCD), panic disorders, eating disorders, anorexia nervosa, bulimia, obesity, narcolepsy, nociception, AIDS-dementia, senile dementia, autism, Parkinson's disease, Huntington's disease, Amyotrophic Lateral Sclerosis, anxiety, non-OCD anxiety disorders, convulsive disorders, epilepsy, neurodegenerative disorders, transient anoxia, induced neuro-degeneration, neuropathy, diabetic neuropathy, peripheral dyslexia, tardive dyskinesia, hyperkinesia, pain, pain of acute, chronic or recurrent character, pain caused by migraine, postoperative pain, phantom limb pain, inflammatory pain, neuropathic pain, chronic headache, central pain, pain related to diabetic neuropathy, pain related to postherpetic neuralgia, pain related to peripheral nerve injury, post-traumatic syndrome, social phobia, sleeping disorders, pseudodementia, Ganser's syndrome, pre-menstrual syndrome, late luteal phase syndrome, chronic fatigue syndrome, mutism, trichotillomania, jet-lag, arrhythmias, smooth muscle contractions, angina pectoris, premature labour, diarrhoea, asthma, tardive dyskinesia, hyperkinesia, premature ejaculation, erectile difficulty, hypertension, inflammatory disorders, inflammatory skin disorders, acne, rosacea, Crohn's disease, inflammatory bowel disease, ulcerative colitis, diarrhoea, withdrawal symptoms caused by termination of use of a nicotine containing product, opioids, heroin, cocaine, morphine, benzodiazepines and benzodiazepine-like drugs, or alcohol.

13. A diaza-bicyclo-alkane derivative of any one of claims 1-9, or a pharmaceutically acceptable addition salt thereof, for use as a pharmaceutical composition/medicament.

14. A diaza-bicyclo-alkane derivative of any one of claims 1-9, or a pharmaceutically acceptable addition salt thereof, for use in the treatment, prevention or alleviation of a disease or a disorder or a condition of a mammal, including a human, which disease, disorder or condition is responsive to modulation of cholinergic receptors and/or monoamine receptors.

## Patentansprüche

1. Diazabicycloalkanderivat, das durch Formel I wiedergegeben ist 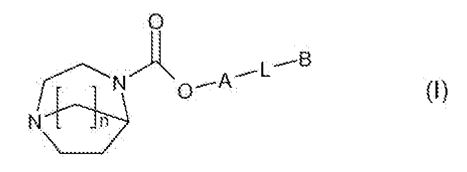 ein Isomer davon oder eine Mischung von seinen Isomeren oder ein pharmazeutisch verträgliches Salz davon, wobei
n 1, 2 oder 3 ist;
A eine Phenyl-, Thiadiazolyl-, Pyridyl- oder Pyridazinylgruppe bedeutet;
B eine Phenyl- oder eine Indolylgruppe bedeutet; wobei diese Phenyl- und Indolylgruppe gegebenenfalls mit Hydroxy oder Alkoxy substituiert sein kann; und
L eine Verbindungsgruppe ausgewählt aus -C=C- und -NHCO- bedeutet.

2. Diazabicycloalkanderivat nach Anspruch 1, wobei n 1 oder 2 ist.

3. Diazabicycloalkanderivat nach Anspruch 1, wobei n 2 ist.

4. Diazabicycloalkanderivat nach Anspruch 3, wobei A eine Phenylgruppe bedeutet.

5. Diazabicycloalkanderivat nach einem der Ansprüche 1-4, wobei B eine Phenylgruppe bedeutet, wobei dieses Phenyl gegebenenfalls mit Hydroxy oder Alkoxy substituiert sein kann.

6. Diazabicycloalkanderivat nach einem der Ansprüche 1-4, wobei B eine Indolylgruppe bedeutet.

7. Diazabicycloalkanderivat nach einem der Ansprüche 1-6, wobei L -C=C- bedeutet.

8. Diazabicycloalkanderivat nach einem der Ansprüche 1-7, wobei L -NHCO- bedeutet.

9. Diazabicycloalkanderivat nach Anspruch 1, bei dem es sich um
1,4-Diaza-bicyclo[3.2.2]nonan-4-carbonsäure-4-phenylethinyl-phenylester;
1,4-Diaza-bicyclo[3.2.2] nonan-4-carbonsäure-4-benzoylamino-phenylester;
1,4-Diaza-bicyclo[3.2.2] nonan-4-carbonsäure-4-(4-methoxy-benzoyl-amino)-phenylester;
1,4-Diaza-bicyclo[3.2.2] nonan-4-carbonsäure-4-(3-methoxy-benzoyl-amino)-phenylester; oder
1,4-Diaza-bicyclo[3.2.2]nonan-4-carbonsäure-4-(2-methoxy-benzoyl-amino)-phenylester handelt;
oder ein Enantiomer oder eine Mischung von seinen Enantiomeren oder ein pharmazeutisch verträgliches Salz davon.

10. Pharmazeutische Zusammensetzung umfassend eine therapeutisch wirksame Menge eines Diazabicycloalkanderivats nach einem der Ansprüche 1-9 oder eines pharmazeutisch verträglichen Additionssalzes davon zusammen mit wenigstens einem pharmazeutisch verträglichen Träger oder Verdünnungsmittel.

11. Verwendung eines Diazabicycloalkanderivats nach einem der Ansprüche 1-9 oder eines pharmazeutisch verträglichen Additionssalzes davon für die Herstellung einer pharmazeutischen Zusammensetzung/eines Medikaments für die Behandlung, Verhütung oder Linderung einer Krankheit oder einer Störung oder eines Zustands eines Säugers, einschließlich eines Menschen, wobei diese Krankheit, diese Störung oder dieser Zustand auf die Modulation von cholinergen Rezeptoren und/oder Monoaminrezeptoren anspricht.

12. Verwendung nach Anspruch 11, wobei es sich bei der Krankheit oder der Störung oder dem Zustand um eine kognitive Störung, ein Lerndefizit, ein Gedächtnisdefizit oder eine Gedächtnisdysfunktion, die Alzheimer-Krankheit, ein Aufmerksamkeitsdefizit, eine Aufmerksamkeitsdefizit-Hyperaktivitätsstörung (ADHD), das Tourette-Syndrom, eine Psychose, Depression, bipolare Störung, Manie, manische Depression, Schizophrenie, kognitive Defizite oder Aufmerksamkeitsdefizite in Verbindung mit Schizophrenie, Zwangsstörungen (OCD), Panikstörungen, Essstörungen, Anorexia nervosa, Bulimie, Fettleibigkeit, Narkolepsie, Nozizeption, AIDS-Demenz, senile Demenz, Autismus, die Parkinson-Krankheit, Chorea Huntington, amyotrophe Lateralsklerose, Angst, Nicht-OCD-Angststörungen, konvulsive Störungen, Epilepsie, neurodegenerative Störungen, vorübergehende Anoxie, induzierte Neurodegeneration, Neuropathie, diabetische Neuropathie, periphere Dyslexie, tardive Dyskinesie, Hyperkinesie, Schmerzen, Schmerzen von akutem, chronischem oder wiederkehrendem Charakter, Schmerzen, die durch Migräne verursacht sind, postoperative Schmerzen, Phantomschmerzen, entzündliche Schmerzen, neurophatische Schmerzen, chronische Kopfschmerzen, zentrale Schmerzen, Schmerzen in Verbindung mit diabetischer Neuropathie, Schmerzen in Verbindung mit postherpetischer Neuralgie, Schmerzen in Verbindung mit einer peripheren Nervenverletzung, das posttraumatische Syndrom, eine Sozialphobie, Schlafstörungen, Pseudodemenz, das Ganser-Syndrom, das prämenstruelle Syndrom, das Syndrom der späten Lutealphase, das chronische Ermüdungssyndrom, Mutismus, Trichotillomanie, Jetlag, Arrhythmien, Kontraktionen eines glatten Muskels, Angina pectoris, eine Frühgeburt, Diarrhö, Asthma, tardive Dyskinesie, Hyperkinesie, vorzeitige Ejakulation, Erektionsschwierigkeiten, Hochdruck, entzündliche Störungen, entzündliche Hautstörungen, Akne, Rosacea, Morbus Crohn, entzündliche Darmerkrankung, Colitis ulcerosa, Diarrhö, Entzugssymptome, die durch die Beendigung des Gebrauchs von einem nikotinhaltigen Produkt, Opioiden, Heroin, Kokain, Morphin, Benzodiazepinen und benzodiazepinartigen Drogen bzw. Arzneimitteln oder Alkohol verursacht sind, handelt.

13. Diazabicycloalkanderivat nach einem der Ansprüche 1-9 oder ein pharmazeutisch verträgliches Additionssalz davon zur Verwendung als eine pharmazeutische Zusammensetzung/ein Medikament.

14. Diazabicycloalkanderivat nach einem der Ansprüche 1-9 oder ein pharmazeutisch verträgliches Additionssalz davon zur Verwendung bei der Behandlung, Verhütung oder Linderung einer Krankheit oder einer Störung oder eines Zustands eines Säugers, einschließlich eines Menschen, wobei diese Krankheit, diese Störung oder dieser Zustand auf die Modulation von cholinergen Rezeptoren und/oder Monoaminrezeptoren anspricht.

## Revendications

1. Dérivé de diaza-bicyclo-alcane représenté par la formule I 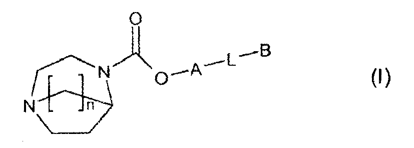 un isomère de celui-ci ou un mélange de ses isomères, ou un sel pharmaceutiquement acceptable de celui-ci, dans lequel
n est 1, 2 ou 3 ;
A représente un groupe phényle, thiadiazolyle, pyridyle ou pyridazinyle ;
B représente un groupe phényle ou indolyle ; lequel groupe phényle et indolyle peut être facultativement substitué par un hydroxy ou un alcoxy ; et
L représente un groupe de liaison choisi parmi -C=C- et -NHCO-.

2. Dérivé de diaza-bicyclo-alcane selon la revendication 1, dans lequel n est 1 ou 2.

3. Dérivé de diaza-bicyclo-alcane selon la revendication 1, dans lequel n est 2.

4. Dérivé de diaza-bicyclo-alcane selon la revendication 3, dans lequel A représente un groupe phényle.

5. Dérivé de diaza-bicyclo-alcane selon l'une quelconque des revendications 1 à 4, dans lequel B représente un groupe phényle ; lequel phényle peut être facultativement substitué par un hydroxy ou un alcoxy.

6. Dérivé de diaza-bicyclo-alcane selon l'une quelconque des revendications 1 à 4, dans lequel B représente un groupe indolyle.

7. Dérivé de diaza-bicyclo-alcane selon l'une quelconque des revendications 1 à 6, dans lequel L représente -C≡C-.

8. Dérivé de diaza-bicyclo-alcane selon l'une quelconque des revendications 1 à 7, dans lequel L représente -NHCO-.

9. Dérivé de diaza-bicyclo-alcane selon la revendication 1, qui est
l'ester 4-phényléthynyl-phénylique de l'acide 1,4-diaza-bicyclo[3.2.2]-nonane-4-carboxylique ;
l'ester 4-benzoylamino-phénylique de l'acide 1,4-diaza-bicyclo[3.2.2]-nonane-4-carboxylique ;
l'ester 4-(4-méthoxy-benzoyl-amino)-phénylique de l'acide 1,4-diaza-bicyclo[3.2.2]nonane-4-carboxylique ;
l'ester 4-(3-méthoxy-benzoyl-amino)-phénylique de l'acide 1,4-diaza-bicyclo[3.2.2]nonane-4-carboxylique ; ou
l'ester 4-(2-méthoxy-benzoyl-amino)-phénylique de l'acide 1,4-diaza-bicyclo[3.2.2]nonane-4-carboxylique ;
ou un énantiomère ou un mélange de ses énantiomères, ou un sel pharmaceutiquement acceptable de celui-ci.

10. Composition pharmaceutique comprenant une quantité thérapeutiquement efficace d'un dérivé de diaza-bicyclo-alcane selon l'une quelconque des revendications 1 à 9, ou d'un sel d'addition pharmaceutiquement acceptable de celui-ci, conjointement avec au moins un support ou diluant pharmaceutiquement acceptable.

11. Utilisation d'un dérivé de diaza-bicyclo-alcane selon l'une quelconque des revendications 1 à 9, ou d'un sel d'addition pharmaceutiquement acceptable de celui-ci, pour la fabrication d'une composition pharmaceutique/médicament pour le traitement, la prévention ou l'atténuation d'une maladie ou d'un trouble ou d'une affection d'un mammifère, incluant un être humain, laquelle maladie, lequel trouble ou laquelle affection réagit à une modulation des récepteurs cholinergiques et/ou des récepteurs des monoamines.

12. Utilisation selon la revendication 11, dans laquelle la maladie ou un trouble ou une affection est un trouble cognitif, un défaut d'apprentissage, une déficience ou un dysfonctionnement de la mémoire, une maladie d'Alzheimer, une déficience de l'attention, un trouble d'hyperactivité avec déficit de l'attention (THADA), un syndrome de Tourette, une psychose, une dépression, un trouble bipolaire, une manie, une manie-dépression, une schizophrénie, des déficiences cognitives ou de l'attention liées à une schizophrénie, des troubles obsessivo-compulsifs (TOC), des troubles paniques, des troubles de l'alimentation, une anorexie mentale, une boulimie, une obésité, une narcolepsie, une nociception, une démence liée au SIDA, une démence sénile, un autisme, une maladie de Parkinson, une maladie de Huntington, une sclérose latérale amyotrophique, une anxiété, des troubles d'anxiété non TOC, des troubles convulsifs, une épilepsie, des troubles neurodégénératifs, une anoxie transitoire, une neurodégénérescence induite, une neuropathie, une neuropathie diabétique, une dyslexie périphérique, une dyskinésie tardive, une hyperkinésie, une douleur, une douleur de caractère aigu, chronique ou récurrent, une douleur provoquée par une migraine, une douleur postopératoire, une douleur de membre fantôme, une douleur inflammatoire, une douleur neuropathique, une céphalée chronique, une douleur centrale, une douleur liée à une neuropathie diabétique, une douleur liée à une névralgie postzostérienne, une douleur liée à une blessure de nerf périphérique, un syndrome post-traumatique, une phobie sociale, des troubles du sommeil, une pseudo-démence, un syndrome de Ganser, un syndrome prémenstruel, un syndrome de phase lutéale tardive, un syndrome de fatigue chronique, un mutisme, une trichotillomanie, un décalage horaire, des arythmies, des contractions des muscles lisses, une angine de poitrine, un travail prématuré, une diarrhée, un asthme, une dyskinésie tardive, une hyperkinésie, une éjaculation prématurée, une difficulté d'érection, une hypertension, des troubles inflammatoires, des troubles cutanés inflammatoires, une acné, une rosacée, une maladie de Crohn, une maladie intestinale inflammatoire, une rectocolite hémorragique, une diarrhée, des symptômes de sevrage provoqués par l'arrêt d'utilisation d'un produit contenant de la nicotine, d'opioïdes, d'héroïne, de cocaïne, de morphine, de benzodiazépines et de substances médicamenteuses de type benzodiazépine, ou d'alcool.

13. Dérivé de diaza-bicyclo-alcane selon l'une quelconque des revendications 1 à 9, ou un sel d'addition pharmaceutiquement acceptable de celui-ci, pour une utilisation en tant que composition pharmaceutique/médicament.

14. Dérivé de diaza-bicyclo-alcane selon l'une quelconque des revendications 1 à 9, ou un sel d'addition pharmaceutiquement acceptable de celui-ci, pour une utilisation dans le traitement, la prévention ou l'atténuation d'une maladie ou d'un trouble ou d'une affection d'un mammifère, incluant un être humain, laquelle maladie, lequel trouble ou laquelle affection réagit à une modulation des récepteurs cholinergiques et/ou des récepteurs des monoamines.
